# EUROPEAN PATENT APPLICATION

(11) **EP 2 124 050 A1**
(43) Date of publication of application: **25.11.2009**
(21) Application number: 08721617.2
(22) Date of filing: 07.03.2008
(51) Int. Cl.: G01N 33/14, C12Q 1/00, C12C 7/00

(54) **METHOD OF EVALUATING FOAM-HOLDING PROPERTY OF FERMENTED MALT DRINK AND MARKER FOR EVALUATING FOAM-HOLDING**

(30) Priority: 07.03.2007 JP 2007057637
(71) Applicant: Sapporo Breweries Limited, Tokyo 150-8522 (JP)
(72) Inventor: IIMURE, Takashi, Tokyo 150-8522 (JP); TAKOI, Kiyoshi, Tokyo 150-8522 (JP); ITO, Kazutoshi, Tokyo 150-8522 (JP); TAKEDA, Kazuyoshi, Kurashiki-shi Okayama 710-0031 (JP)
(74) Representative: Vossius & Partner
(86) International application number: PCT/JP2008/054200
(87) International publication number: WO 2008/111527

(57) **Abstract**

The present invention provides a method for determination of the foam stability of a fermented malt beverage, wherein the concentration of yeast thioredoxin (M_{T}) in the fermented malt beverage or a fermenting material solution of the fermented malt beverage is used as an index having a negative correlation with the foam stability of the fermented malt beverage, and the concentration of protein Z (M_{Z}) in the fermented malt beverage or a pre-fermentation or fermenting material solution of the fermented malt beverage is used as an index having a positive correlation with the foam stability of the fermented malt beverage, to determine the foam stability of the fermented malt beverage. According to the determination method of the invention, it is possible to determine the foam stability from the concentration of prescribed proteins in the fermented malt beverage or pre-fermentation or fermenting material solution thereof without directly measuring the head retention of the fermented malt beverage.

## Description

### Technical Field

The present invention relates to a method for determination of the foam stability of a fermented malt beverage and to a marker for determination of foam stability.

### Background Art

Foam on fermented malt beverages such as beer is an aggregate of carbon dioxide gas bubbles surrounded by thin liquid films. It acts as a lid to prevent loss of carbonation and alteration of flavor, while also enhancing the aroma. The foam stability of fermented malt beverages is therefore one of the important factors for judging the quality of fermented malt beverages, and research has been conducted to improve the foam stability of fermented malt beverages.

The foam stability of a fermented malt beverage has been determined based on the NIBEM value, i.e. the time (seconds) required for the foam to collapse over a distance of 30 mm after the fermented malt beverage at 20°C has been poured into a standard glass with a foam dispenser.

LTP-1 and hordein have been reported as barley proteins associated with the foam stability of fermented malt beverages (Non-patent documents 1 and 2).

Recently, research using proteomics has been conducted to comprehensively analyze proteins based on genomic information. Also as for barley to be used as a material for fermented malt beverages, attempts have been made to identify proteins associated with malt qualities by comparing the malt qualities with two-dimensional gel electrophoresis patterns (Non-patent documents 3 to 5).
Non-patent document 1: Bamforth et al., 2004, J. Sci. Food Agric., Vol. 84, p.1001-1004
Non-patent document 2: Van Nierop et al., 2004, J. Agric. Food Chem., Vol. 52, p.3120-3129
Non-patent document 3: Ostergaard et al., 2004, Proteomics, Vol. 4, p.2437-2447
Non-patent document 4: Sass Bak-Jensen et al., 2004, Proteomics, Vol. 4, p.728-742
Non-patent document 5: Finnie and Svensson, 2004, Proc. 9th International Barley Genetics Symposium, p.431-436

### Disclosure of the Invention

### Problem to be Solved by the Invention

The NIBEM value, which is used as a criterion for determining the foam stability of a fermented malt beverage, is greatly affected by temperature, weather and artificial factors, and it is difficult to determine the foam stability by comparing NIBEM values measured under different conditions.

Also, although LTP-1 and hordein in barley have been reported to be associated with the foam stability of a fermented malt beverage, the correlation with the NIBEM value has not been elucidated, and these have not been used as generally applicable markers for determination of foam stability.

In addition, although proteomic studies of barley have been promoted, no new proteins associated with the foam stability of a fermented malt beverage have been discovered.

It is an object of the present invention to provide a method for determination of the foam stability of a fermented malt beverage, which makes it possible to determine the foam stability from the concentration of prescribed proteins in the fermented malt beverage or pre-fermentation or fermenting material solution thereof without directly measuring the head retention of the fermented malt beverage. It is another object of the present invention to provide a marker for determination of the foam stability of a fermented malt beverage.

### Means for Solving the Problem

In order to achieve the objects stated above, the present invention provides a method for determination of the foam stability of a fermented malt beverage, wherein the concentration of yeast thioredoxin (M_{T}) in the fermented malt beverage or a fermenting material solution of the fermented malt beverage is used as an index having a negative correlation with the foam stability of the fermented malt beverage, and the concentration of protein Z (M_{Z}) in the fermented malt beverage or a pre-fermentation or fermenting material solution of the fermented malt beverage is used as an index having a positive correlation with the foam stability of the fermented malt beverage, to determine the foam stability of the fermented malt beverage.

The present inventors comprehensively analyzed proteins in beer using proteomics as a tool, and found that in the case of a beer with high NIBEM value and good head retention, the yeast thioredoxin concentration is low and the protein Z concentration and barley dimeric alpha-amylase inhibitor (hereinafter "BDAI-1") concentration are high. Also, as a result of statistical analysis of the multiple correlation of the yeast thioredoxin and protein Z concentrations with the NIBEM value, the present inventors found that these concentrations can be used for determination of the foam stability of a fermented malt beverage.

In the determination method defined above, measurements of the concentrations of yeast thioredoxin and protein Z in the fermented malt beverage etc. make it possible to determine the foam stability without measuring the NIBEM value. Since measurements of the yeast thioredoxin and protein Z concentrations can be performed without the effects of temperature, weather and artificial factors, it allows more generally applicable and more accurate determination of the foam stability than measurement of the NIBEM value.

In the determination method defined above, it is preferred, for example, that the value of the formula: a - b × M_{T} + c × M_{Z} [where a is a positive or negative number or 0, and b and c are positive numbers] be used as an index having a positive correlation with the foam stability of the fermented malt beverage, to determine the foam stability of the fermented malt beverage. The value can be used as a value having a linear relationship with the NIBEM value, to determine the foam stability of the fermented malt beverage. If a is 0, the determination can be performed more conveniently.

Also, it is preferred that a, b and c in the formula: a - b × M_{T} + c × M_{Z} be the partial regression coefficients of a predictive multiple regression equation for the NIBEM value of the fermented malt beverage as a function of M_{T} and M_{Z}.

In this case, the NIBEM value can be predicted from the yeast thioredoxin concentration (M_{T}) and protein Z concentration (M_{Z}) without directly measuring the NIBEM value using a specialized apparatus, standard glass, etc. for measurement of the NIBEM value. The foam stability measured by this determination method can be compared with the NIBEM value that has already been measured by a conventional method.

In the determination method defined above, it is preferred that the concentration of BDAI-1 (M_{B}) in the fermented malt beverage or a pre-fermentation or fermenting material solution of the fermented malt beverage be further used as an index having a positive correlation with the foam stability of the fermented malt beverage, to determine the foam stability of the fermented malt beverage.

If, in addition to the yeast thioredoxin concentration in the fermented malt beverage or a fermenting material solution thereof and the protein Z concentration in the fermented malt beverage or a prefermentation or fermenting material solution thereof, the BDAI-1 concentration in the fermented malt beverage or a pre-fermentation or fermenting material solution thereof is used as an index relating to the foam stability, it is possible to more accurately determine the foam stability of the fermented malt beverage.

In the determination method defined above, it is preferred, for example, that the value of the formula: a - b × M_{T} + c × M_{Z} + d × M_{B} [where a is a positive or negative number or 0, and b, c and d are positive numbers] be used as an index having a positive correlation with the foam stability of the fermented malt beverage, to determine the foam stability of the fermented malt beverage. If a is 0, the determination can be performed more conveniently.

Also, it is preferred that a, b, c and d in the formula: a - b × M_{T} + c × M_{Z} + d × M_{B} be the partial regression coefficients of a predictive multiple regression equation for the NIBEM value of the fermented malt beverage as a function of M_{T}, M_{Z} and M_{B}.

In this case, the NIBEM value can be more accurately predicted from the yeast thioredoxin concentration (M_{T}), protein Z concentration (M_{Z}) and BDAI-1 concentration (M_{B}) without directly measuring the NIBEM value using a specialized apparatus, standard glass, etc. for measurement of the NIBEM value, and it can be compared with the NIBEM value that has already been measured by a conventional method.

The present invention also provides a marker for determination of the foam stability of a fermented malt beverage, the marker being composed of yeast thioredoxin.

Yeast thioredoxin can be considered a negative factor for the foam stability of a fermented malt beverage. Since a high concentration of yeast thioredoxin in a fermented malt beverage or a fermenting material solution of a fermented malt beverage impairs the foam stability of the fermented malt beverage, it can be used as a marker for determination of foam stability and also as a selection marker for a yeast strain to be used in the production of a fermented malt beverage with improved foam stability.

The present invention further provides a marker for determination of the foam stability of a fermented malt beverage, the marker being composed of BDAI-1.

BDAI-1 can be considered a positive factor for the foam stability of a fermented malt beverage. Since a high concentration of BDAI-1 in a fermented malt beverage or a pre-fermentation or fermenting material solution of a fermented malt beverage improves the foam stability of the fermented malt beverage, it can be used as a marker for determination of foam stability and also as a selection marker for a barley variety to be used in the production of a fermented malt beverage with improved foam stability.

### Effects of the Invention

According to the present invention, it is possible to determine the foam stability and also predict the NIBEM value without directly measuring the NIBEM value. Also, the method for determination of foam stability according to the invention can be carried out without the effects of temperature, weather and artificial factors, and therefore it allows more generally applicable and more accurate determination of the foam stability than measurement of the NIBEM value.

In addition, the marker for determination of foam stability according to the invention can be used as a selection marker for a yeast strain or barley variety to be used in the production of a fermented malt beverage with improved foam stability.

### Brief Description of the Drawings

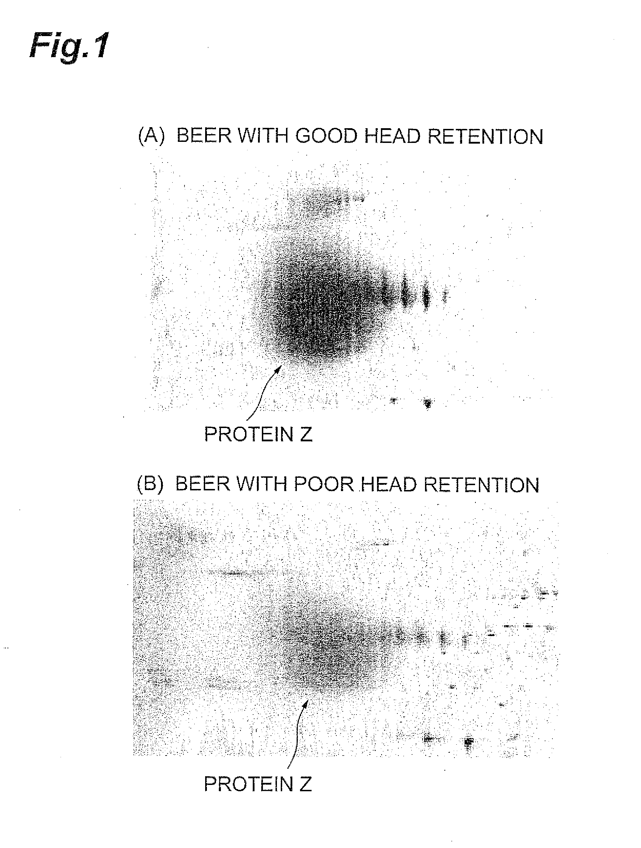

Fig. 1 is a pair of photographs of two-dimensional gel electrophoresis showing the spots corresponding to protein Z from a beer with good head retention and a beer with poor head retention. A significant difference was found in spot intensity between the two beers, and the spot intensity was higher for the beer with good head retention.

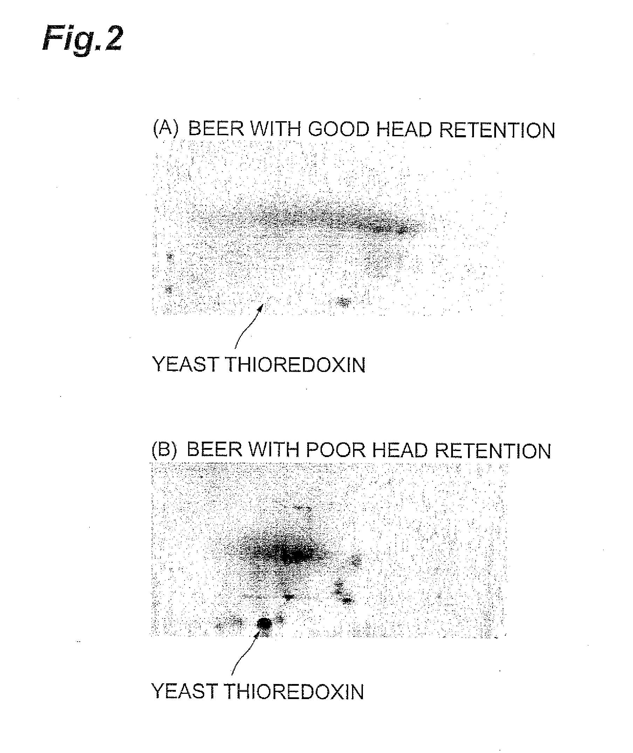

Fig. 2 is a pair of photographs of two-dimensional gel electrophoresis showing the spots corresponding to yeast thioredoxin from a beer with good head retention and a beer with poor head retention. A significant difference was found in spot intensity between the two beers, and the spot intensity was lower for the beer with good head retention.

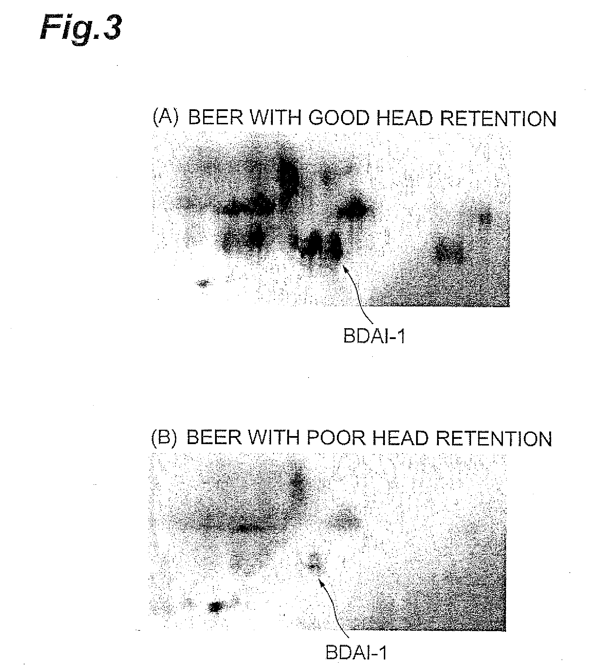

Fig. 3 is a pair of photographs of two-dimensional gel electrophoresis showing the spots corresponding to BDAI-1 from a beer with good head retention and a beer with poor head retention. A significant difference was found in spot intensity between the two beers, and the spot intensity was higher for the beer with good head retention.

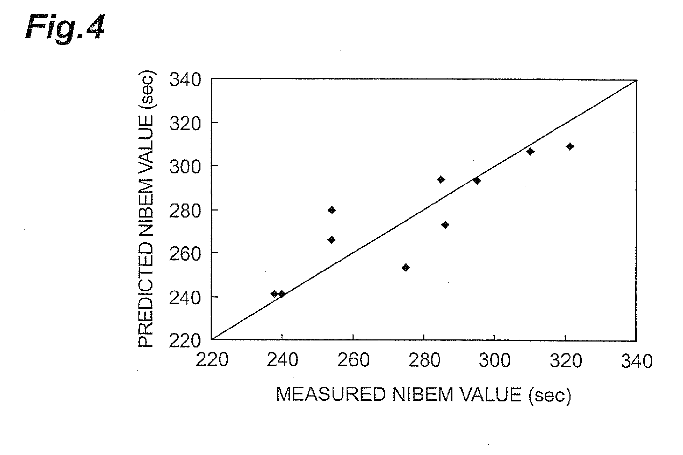

Fig. 4 is a graph showing a relationship between the predicted NIBEM value and measured NIBEM value, the relationship being obtained when using the NIBEM value as the response variable and the protein Z concentration and yeast thioredoxin concentration as the explanatory variables.

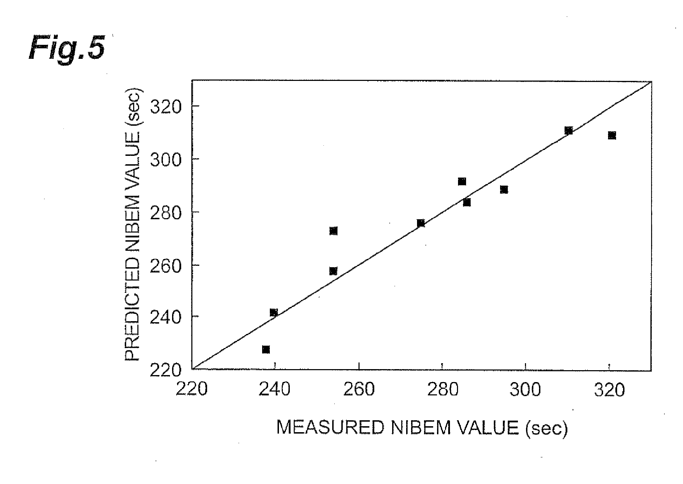

Fig. 5 is a graph showing a relationship between the predicted NIBEM value and measured NIBEM value, the relationship being obtained when using the NIBEM value as the response variable and the protein Z concentration, yeast thioredoxin concentration and BDAI-1 concentration as the explanatory variables.

### Best Modes for Carrying Out the Invention

Preferred embodiments of the present invention will now be described in detail.

The determination method of the invention is a method for determination of the foam stability of a fermented malt beverage, wherein the concentration of yeast thioredoxin (M_{T}) in the fermented malt beverage or a fermenting material solution of the fermented malt beverage is used as an index having a negative correlation with the foam stability of the fermented malt beverage, and the concentration of protein Z (M_{Z}) in the fermented malt beverage or a pre-fermentation or fermenting material solution of the fermented malt beverage is used as an index having a positive correlation with the foam stability of the fermented malt beverage, to determine the foam stability of the fermented malt beverage.

As used herein, "head retention" refers to the time required for disappearance of the foam produced when pouring a fermented malt beverage into a glass. If the required time is long, the "head retention" is "good". If the required time is short, the "head retention" is "poor". "Foam stability" means the extent to which the head retention is good or poor, and it is a basis for comparing the head retention of a fermented malt beverage with that of another fermented malt beverage. A "method for determination of foam stability" is a method for determining whether the head retention of a fermented malt beverage is good or poor compared to another fermented malt beverage.

A "fermented malt beverage" is a beverage brewed using malt as a material, and as examples there may be mentioned beer, low-malt beer (happoshu), and other types of alcoholic beverages obtained using malt as a material. Beer is a fermented beverage obtained using malt, hops and water as materials or using malt, hops, water, and barley or another commodity established by the Japanese government ordinance (barley, rice, corn, kaoliang, potato, starch, saccharide, or a bittering agent or coloring agent approved by the Department of the Treasury) as materials, with the proportion of malt used being 2/3 or greater. Low-malt beer (happoshu) is an effervescent alcoholic beverage obtained using malt or barley as part of the materials, with the proportion of malt used being less than 2/3.

A "pre-fermentation material solution of a fermented malt beverage" is: the material solution that exists from the time the malt is subjected to mashing until wort is produced from the malt; the wort that exists before boiling; the wort that exists from the time the wort is boiled until it is cooled to yield cold wort to which yeast is to be added; or the cold wort. A "fermenting material solution of a fermented malt beverage" is the fermentate that exists from the time yeast is added to the cold wort, which is then subjected to fermentation, until a fermented malt beverage is obtained.

"Yeast thioredoxin" is an oxidoreductase, and it is a yeast protein that is eluted from yeast cells into the fermented malt beverage (NCBI Accession NP_011725).

The yeast thioredoxin concentration (M_{T}) in a fermented malt beverage can be measured using, for example: ELISA; Western blotting; image analysis of the stained region (hereinafter "spot") obtained by staining of the protein fractionated by two-dimensional gel electrophoresis; protein chip technology; or affinity chromatography-based quantitative analysis.

The anti-yeast thioredoxin antibody to be used in ELISA or Western blotting may be any antibody that can specifically recognize yeast thioredoxin from a yeast to be used for fermentation. For example, it can be prepared by immunizing a rabbit, mouse or the like using as antigen a peptide synthesized based on the amino acid sequence of yeast thioredoxin.

In image analysis of the spot obtained by staining of the protein fractionated by two-dimensional gel electrophoresis, the yeast thioredoxin concentration can be estimated by imaging the spot corresponding to yeast thioredoxin, quantifying the staining intensity per unit area (for example, based on pixel intensity), and then summing the values for the entire spot.

"Protein Z" is a serine protease inhibitor present in barley seeds and beer, and the term collectively refers to the two proteins protein Z4 and protein Z7. Both protein Z4 (NCBI Accession CAA66232) and protein Z7 (NCBI Accession CAA64599) belong to the same subfamily of barley serine protease inhibitor, and their amino acid sequences have approximately 73% homology to each other.

The concentration of protein Z (M_{Z}) in a fermented malt beverage or a pre-fermentation or fermenting material solution of the fermented malt beverage can be measured using, for example: ELISA; Western blotting; image analysis of the spot obtained by staining of the protein fractionated by two-dimensional gel electrophoresis; protein chip technology; or affinity chromatography-based quantitative analysis.

The anti-protein Z antibody to be used in ELISA or Western blotting is preferably an antibody that can recognize both protein Z4 and protein Z7 from a barley to be used for fermentation. For example, it can be prepared by immunizing a rabbit, mouse or the like using as antigen a peptide synthesized based on the common region of the amino acid sequences of protein Z4 and protein Z7. When an antibody that can recognize both protein Z4 and protein Z7 is not available, the protein Z concentration may be estimated from the sum of the protein Z4 and protein Z7 concentrations. The protein Z4 can be quantitated with an antibody that specifically recognizes only protein Z4, and the protein Z7 can be quantitated with an antibody that specifically recognizes only protein Z7.

In image analysis of the spot obtained by staining of the protein fractionated by two-dimensional gel electrophoresis, the protein Z concentration can be estimated by imaging the spot corresponding to protein Z, quantifying the staining intensity per unit area (for example, based on pixel intensity), and then summing the values for the entire spot.

In the determination method defined above, it is preferred, for example, that the value of the formula: a - b × M_{T} + c × M_{Z} [where a is a positive or negative number or 0, and b and c are positive numbers] be used as an index having a positive correlation with the foam stability of the fermented malt beverage, to determine the foam stability of the fermented malt beverage. The value can be used as a value having a linear relationship with the NIBEM value, to determine the foam stability of the fermented malt beverage. If a is 0, the determination can be performed more conveniently.

Also, it is preferred that a, b and c in the formula: a - b × M_{T} + c × M_{Z} be the partial regression coefficients of a predictive multiple regression equation for the NIBEM value of the fermented malt beverage as a function of M_{T} and M_{Z}.

The partial regression coefficients of a predictive multiple regression equation for the NIBEM value of a fermented malt beverage can be obtained by: measuring the concentration of yeast thioredoxin (M_{T}) in the fermented malt beverage or a fermenting material solution of the fermented malt beverage and the concentration of protein Z (M_{Z}) in the fermented malt beverage or a pre-fermentation or fermenting material solution of the fermented malt beverage, for a plurality of fermented malt beverages whose NIBEM values have been measured, and then performing multiple regression analysis using the NIBEM value of the fermented malt beverage as the response variable and the yeast thioredoxin concentration (M_{T}) and protein Z concentration (M_{Z}) as the explanatory variables.

In the determination method defined above, it is preferred that the concentration of BDAI-1 (M_{B}) in the fermented malt beverage or a pre-fermentation or fermenting material solution of the fermented malt beverage be further used as an index having a positive correlation with the foam stability of the fermented malt beverage, to determine the foam stability of the fermented malt beverage.

"BDAI-1", or barley dimeric alpha-amylase inhibitor (NCBI Accession CAA08836), is known to inhibit insect α-amylase but not barley α-amylase (Mena et al., 1992, Plant Mol. Biol., Vol. 20, p.451-458).

The BDAI-1 concentration (M_{B}) in a fermented malt beverage can be measured using, for example: ELISA; Western blotting; image analysis of the spot obtained by staining of the protein fractionated by two-dimensional gel electrophoresis; protein chip technology; or affinity chromatography-based quantitative analysis.

The anti-BDAI-1 antibody to be used in ELISA or Western blotting may be any antibody that can specifically recognize BDAI-1 from a malt or barley to be used for fermentation. For example, it can be prepared by immunizing a rabbit, mouse or the like using as antigen a peptide synthesized based on the amino acid sequence of BDAI-1.

In image analysis of the spot obtained by staining of the protein fractionated by two-dimensional gel electrophoresis, the BDAI-1 concentration can be estimated by imaging the spot corresponding to BDAI-1, quantifying the staining intensity per unit area (for example, based on pixel intensity), and then summing the values for the entire spot.

In the determination method defined above, it is preferred, for example, that the value of the formula: a - b × M_{T} + c × M_{Z} + d × M_{B} [where a is a positive or negative number or 0, and b, c and d are positive numbers] be used as an index having a positive correlation with the foam stability of the fermented malt beverage, to determine the foam stability of the fermented malt beverage. If a is 0, the determination can be performed more conveniently.

Also, it is preferred that a, b, c and d in the formula: a - b × M_{T} + c × M_{Z} + d × M_{B} be the partial regression coefficients of a predictive multiple regression equation for the NIBEM value of the fermented malt beverage as a function of M_{T}, Mz and M_{B}.

The partial regression coefficients of a predictive multiple regression equation for the NIBEM value of a fermented malt beverage can be obtained by: measuring the concentration of yeast thioredoxin (M_{T}) in the fermented malt beverage or a fermenting material solution of the fermented malt beverage and the concentrations of protein Z (M_{Z}) and BDAI-1 (M_{B}) in the fermented malt beverage or a pre-fermentation or fermenting material solution of the fermented malt beverage, for a plurality of fermented malt beverages whose NIBEM values have been measured, and then performing multiple regression analysis using the NIBEM value of the fermented malt beverage as the response variable and the yeast thioredoxin concentration (M_{T}), protein Z concentration (M_{Z}) and BDAI-1 concentration (M_{B}) as the explanatory variables.

The marker for determination of foam stability according to the invention is characterized by being composed of yeast thioredoxin or BDAI-1.

Here, a "marker for determination of foam stability" is a protein which can serve as an index for determining or predicting the foam stability of a fermented malt beverage, wherein the concentration of the protein in the fermented malt beverage or a pre-fermentation or fermenting material solution of the fermented malt beverage shows a correlation with the NIBEM value. It has never been known that yeast thioredoxin and BDAI-1 can be utilized for the determination or prediction of the foam stability of a fermented malt beverage.

Yeast thioredoxin can be considered a negative factor for the foam stability of a fermented malt beverage. Since a high concentration of yeast thioredoxin in a fermented malt beverage or a fermenting material solution of a fermented malt beverage impairs the foam stability of the fermented malt beverage, it can be used as a marker for determination of foam stability and also as a selection marker for a yeast strain to be used in the production of a fermented malt beverage with improved foam stability. On the other hand, BDAI-1 can be considered a positive factor for the foam stability of a fermented malt beverage. Since a high concentration of BDAI-1 in a fermented malt beverage or a pre-fermentation or fermenting material solution of a fermented malt beverage improves the foam stability of the fermented malt beverage, it can be used as a marker for determination of foam stability and also as a selection marker for a barley variety to be used in the production of a fermented malt beverage with improved foam stability.

Yeast thioredoxin is eluted from yeast cells used for fermentation into the fermented malt beverage, and it can be quantitated using, for example: ELISA; Western blotting; image analysis of the spot obtained by staining of the protein fractionated by two-dimensional gel electrophoresis; protein chip technology; or affinity chromatography-based quantitative analysis.

BDAI-1 is eluted from malt or barley into the fermented malt beverage or a pre-fermentation or fermenting material solution of the fermented malt beverage, and it can be quantitated using, for example: ELISA; Western blotting; image analysis of the spot obtained by staining of the protein fractionated by two-dimensional gel electrophoresis; protein chip technology; or affinity chromatography-based quantitative analysis.

### Examples

The present invention will now be explained in greater detail based on examples and comparative examples. However, the present invention is not limited to the examples described below.

### [Example 1: Identification of proteins having an effect on the head retention of beer]

Proteins in a beer with good head retention and proteins in a beer with poor head retention were fractionated by two-dimensional gel electrophoresis. Image analysis of the stained regions ("spots") detected by silver staining of proteins in the gel was performed, and the proteins that showed a significant difference in concentration between the beer with good head retention and beer with poor head retention were identified by mass spectrometry. This will now be explained in detail.

First, the beer with good head retention (NIBEM value: 275 to 321) and beer with poor head retention (NIBEM value: 238 to 254) were degassed. After measuring the protein concentration by the Bradford method, 3 mL of each beer was desalted by applying it to a PD-10 column (Amersham Biosciences) that had been equilibrated with Milli-Q water, and then eluting the column with 4 mL of Milli-Q water. The protein concentration in each desalted beer sample thus obtained was measured again by the Bradford method, and all of each sample was freeze-dried.

Then, each freeze-dried beer sample was dissolved in a loading buffer (8M urea, 2% CHAPS, 0.28% DTT), and the proteins (100 µg) in each beer sample were fractionated by two-dimensional gel electrophoresis. The two-dimensional gel electrophoresis was performed using a Multiphor II system (Amersham Biosciences), according to the manufacturer's protocol.

After the two-dimensional gel electrophoresis, the fractionated proteins in the gel were silver stained using a Silver Staining Kit, Protein (Amersham Biosciences). With respect to mass spectrometric analysis of the spots that showed a large difference in protein concentration between the beer with good head retention and beer with poor head retention, silver staining was performed using a Silver Stain MS Kit (Wako).

The silver stained gel was subjected to image analysis using ImageMaster 2-D Platinum (Amersham Biosciences). The protein concentration in each spot (hereinafter "spot intensity") was estimated by quantifying the staining intensity of each spot in units of vol% using a densitometer, and then multiplying this value by the protein concentration in the beer sample as measured by the Bradford method before desalting.

With respect to the spots that showed a large difference in the spot intensity estimated in the image analysis between the beer with good head retention and beer with poor head retention, the gel spots were cut out from the gel, and were destained by immersion in a destaining solution contained in the Silver Stain MS Kit (Wako). Tris buffer (pH 8.0) containing trypsin was added to the destained gel spots, which were subsequently subjected to enzyme treatment at 35°C for 20 hours. The digested proteins were then eluted from the gel. After desalting, they were naturally dried, and subjected to MALDI-TOF MS (Voyager-DE STR, Applied Biosystems). An MS-Fit search was performed against the NCBI nr and BaEST020808 protein databases.

Mass spectrometry of yeast thioredoxin was performed as follows. Tris buffer (pH 8.0) containing lysyl endopeptidase was added to the destained gel spot, which was subsequently subjected to enzyme treatment at 35°C for 3 hours. Further, trypsin was added and enzyme treatment at 35°C for 20 hours was performed. Then, the sample solution was analyzed by LC-MS/MS (MAGIC 2002, Michrom Bioresources, USA).

As a result, it was possible to identify the proteins that showed a difference in spot intensity between the beer with good head retention and beer with poor head retention. It was found that only the 3 proteins protein Z, yeast thioredoxin and BDAI-1 contributed to a correlation with the NIBEM value in the multiple regression analyses described in Example 2.

Fig. 1 is a pair of photographs of two-dimensional gel electrophoresis showing the spots corresponding to protein Z from a beer with good head retention and a beer with poor head retention. A significant difference was found in spot intensity between the two beers, and the spot intensity was higher for the beer with good head retention. Fig. 2 is a pair of photographs of two-dimensional gel electrophoresis showing the spots corresponding to yeast thioredoxin from a beer with good head retention and a beer with poor head retention. A significant difference was found in spot intensity between the two beers, and the spot intensity was lower for the beer with good head retention. Fig. 3 is a pair of photographs of two-dimensional gel electrophoresis showing the spots corresponding to BDAI-1 from a beer with good head retention and a beer with poor head retention. A significant difference was found in spot intensity between the two beers, and the spot intensity was higher for the beer with good head retention.

### [Example 2: Multiple regression analysis using the NIBEM value as the response variable and proteins exhibiting a significant difference in spot intensity between a beer with good head retention and a beer with poor head retention as the explanatory variables]

The NIBEM values of 10 commercially available fermented malt beverages (beers and low-malt beers), and the concentrations of the proteins identified in Example 1 in each fermented malt beverage were measured, and simple regression analysis between the NIBEM value and each protein concentration was performed for each fermented malt beverage. As a result, no significant correlations were found.

Multiple regression analysis was therefore performed using two of the protein concentrations as the explanatory variables.

The measurement of the NIBEM value was performed using NIBEM-T, Inpack 2000 and a standard glass for measuring the NIBEM value (Haffmans). Specifically, each of the fermented malt beverages was brought to 20°C and poured into a standard glass with a foam dispenser using carbon dioxide gas. The collapse of the produced foam was followed using the NIBEM-T meter, and the time (seconds) required for the foam to collapse over a distance of 30 mm was recorded as the NIBEM value.

As explained in Example 1, the concentration of each protein was estimated by: fractioning the proteins in the 10 fermented malt beverages by two-dimensional gel electrophoresis, performing image analysis of the silver stained gel using ImageMaster 2-D Platinum, quantifying the staining intensity (vol%) of the intended protein using a densitometer, and then multiplying this value by the protein concentration in the fermented malt beverage as measured by the Bradford method.

As a result, a significant correlation at the 1% level was found only when using the NIBEM value as the response variable and the protein Z concentration and yeast thioredoxin concentration as the explanatory variables. The multiple regression equation was: NIBEM value = 229.93 + (-0.9238 × yeast thioredoxin concentration) + (0.0040 × protein Z concentration), and the standardized multiple regression equation was: NIBEM value = (-0.689 × yeast thioredoxin concentration) + (0.641 × protein Z concentration) (adjusted R² = 0.709).

Fig. 4 is a graph showing the relationship between the predicted NIBEM value and measured NIBEM value, the relationship being obtained when using the NIBEM value as the response variable and the protein Z concentration and yeast thioredoxin concentration as the explanatory variables.

The obtained results demonstrate that protein Z and yeast thioredoxin can be used as markers for determination of the foam stability of a fermented malt beverage. Also, the results demonstrate that protein Z can be used as an index having a positive correlation with the foam stability and yeast thioredoxin can be used as an index having a negative correlation with the foam stability, to determine the foam stability of the fermented malt beverage.

Next, multiple regression analysis was performed using the NIBEM value as the response variable and using the concentration of the protein identified in Example 1, in addition to the protein Z concentration and yeast thioredoxin concentration, as the explanatory variables.

As a result, a significant correlation at the 1% level was found only when using the NIBEM value as the response variable and the protein Z concentration, yeast thioredoxin concentration and BDAI-1 concentration as the explanatory variables. The multiple regression equation was: NIBEM value = 196.74 + (-0.9299 × yeast thioredoxin concentration) + (0.0057 × protein Z concentration) + (0.1169 × BDAI-1 concentration), and the standardized multiple regression equation was: NIBEM value = (-0.694 × yeast thioredoxin concentration) + (0.895 × protein Z concentration) + (0.441 × BDAI-1 concentration) (adjusted R² = 0.856).

Fig. 5 is a graph showing the relationship between the predicted NIBEM value and measured NIBEM value, the relationship being obtained when using the NIBEM value as the response variable and the protein Z concentration, yeast thioredoxin concentration and BDAI-1 concentration as the explanatory variables.

The obtained results demonstrate that BDAI-1 can be used as a marker for determination of the foam stability of a fermented malt beverage. Also, the results demonstrate that the yeast thioredoxin concentration can be used as an index having a negative correlation with the foam stability, and the protein Z concentration and BDAI-1 concentration can be used as indices having a positive correlation with the foam stability, to determine the foam stability of the fermented malt beverage.

## Claims

1. A method for determination of the foam stability of a fermented malt beverage, wherein
the concentration of yeast thioredoxin (M_{T}) in the fermented malt beverage or a fermenting material solution of the fermented malt beverage is used as an index having a negative correlation with the foam stability of the fermented malt beverage, and
the concentration of protein Z (M_{Z}) in the fermented malt beverage or a pre-fermentation or fermenting material solution of the fermented malt beverage is used as an index having a positive correlation with the foam stability of the fermented malt beverage, to determine the foam stability of the fermented malt beverage.

2. The determination method according to claim 1, wherein the value of the formula: a - b × M_{T} + c × M_{Z} [where a is a positive or negative number or 0, and b and c are positive numbers] is used as an index having a positive correlation with the foam stability of the fermented malt beverage, to determine the foam stability of the fermented malt beverage.

3. The determination method according to claim 2, wherein a in the formula: a - b × M_{T} + c × M_{Z} is 0.

4. The determination method according to claim 2, wherein a, b and c in the formula: a - b × M_{T} + c × M_{Z} are the partial regression coefficients of a predictive multiple regression equation for the NIBEM value of the fermented malt beverage as a function of M_{T} and M_{Z}.

5. The determination method according to claim 1, wherein the concentration of barley dimeric alpha-amylase inhibitor (BDAI-1) (M_{B}) in the fermented malt beverage or a pre-fermentation or fermenting material solution of the fermented malt beverage is further used as an index having a positive correlation with the foam stability of the fermented malt beverage, to determine the foam stability of the fermented malt beverage.

6. The determination method according to claim 5, wherein the value of the formula: a - b × M_{T} + c × M_{Z} + d × M_{B} [where a is a positive or negative number or 0, and b, c and d are positive numbers] is used as an index having a positive correlation with the foam stability of the fermented malt beverage, to determine the foam stability of the fermented malt beverage.

7. The determination method according to claim 6, wherein a in the formula: a - b × M_{T} + c × M_{Z} + d × M_{B} is 0.

8. The determination method according to claim 6, wherein a, b, c and d in the formula: a - b × M_{T} + c × M_{Z} + d × M_{B} are the partial regression coefficients of a predictive multiple regression equation for the NIBEM value of the fermented malt beverage as a function of M_{T}, M_{Z} and M_{B}.

9. A marker for determination of the foam stability of a fermented malt beverage, the marker being composed of yeast thioredoxin.

10. A marker for determination of the foam stability of a fermented malt beverage, the marker being composed of barley dimeric alpha-amylase inhibitor (BDAI-1).
